# EUROPÄISCHE PATENTSCHRIFT

(11) **EP 0 701 419 B1**
(45) Veröffentlichungstag und Bekanntmachung des Hinweises auf die Patenterteilung: **27.08.1997**
(21) Anmeldenummer: 94917609.3
(22) Anmeldetag: 10.05.1994
(51) Int. Cl.: A61B 17/58, A61B 17/16

(54) **OSTEOSYNTHESE-HILFSMITTEL ZUR VERSORGUNG SUBTROCHANTERER, PERTROCHANTERER UND SCHENKELHALSFRAKTUREN**
OSTEOSYNTHETIC DEVICE FOR TREATING SUBTROCHANTERIC AND PERTROCHANTERIC FRACTURES AND FRACTURES OF THE NECK OF THE FEMUR
INSTRUMENT AUXILIAIRE POUR OSTEOSYNTHESE PERMETTANT DE TRAITER DES FRACTURES SUBTROCHANTERIENNES ET PERTROCHANTERIENNES, AINSI QUE DES FRACTURES DU COL DU FEMUR

(30) Priorität: 01.06.1993 DE 4318150
(43) Veröffentlichungstag der Anmeldung: 20.03.1996
(60) Teilanmeldung: 96109520.5
(73) Patentinhaber: ENDOCARE AG, CH-6343 Rotkreuz (CH)
(72) Erfinder: FRIEDL, Wilhelm, D-69214 Eppelheim (DE)
(74) Vertreter: Popp, Eugen, Dr.
(86) Internationale Anmeldenummer: EP9401503
(87) Internationale Veröffentlichungsnummer: WO9427508

(56) Entgegenhaltungen:
- EP-A- 0 321 170
- EP-A- 0 486 483
- EP-A- 0 521 600
- EP-A- 0 586 824
- DE-U- 9 102 018
- FR-A- 2 668 360
- GB-A- 2 209 947
- US-A- 3 433 220
- US-A- 3 561 437
- US-A- 4 103 683

## Beschreibung

Die Erfindung betrifft ein Osteosynthese-Hilfsmittel mit einem Verriegelungsnagel, einem Schenkelhalsteil und einem diesem zugeordneten Verriegelungsstift, gemäß dem Oberbegriff des Patentanspruches 1.

Ein derartiges Osteosynthese-Hilfsmittel ist z. B. bekannt aus der EP 0 321 170 A1. Bei diesem bekannten Osteosynthese-Hilfsmittel dient der Verriegelungsnagel zur Führung und Halterung einer Schenkelhalsschraube. Zu diesem Zweck weist der Verriegelungsnagel im proximalen Bereich eine schräge Durchbohrung auf, durch die die Schenkelhalsschraube hindurchführbar ist. Am femurkopfseitigen Ende der Schenkelhalsschraube ist ein selbstschneidendes Gewinde ausgebildet, mit dem die Verankerung im Femurkopf erfolgt. Des weiteren ist in das proximale Ende des Verriegelungsnagels ein Verriegelungsstift einsetzbar, mit dem die Schenkelhalsschraube gegen Drehung gesichert wird. Gleichzeitig dient dieser Verriegelungsstift in Zusammenwirkung mit am Umfang der Schenkelhalsschraube ausgebildeten Axialnuten als Begrenzung für die Axialbewegung der Schenkelhalsschraube innerhalb der schrägen Durchgangsbohrung des Verriegelungsnagels. Wahlweise kann mit dem Verriegelungsstift auch eine starre Verbindung zwischen Schenkelhalsschraube und Verriegelungsnagel hergestellt werden. Bei dieser bekannten Konstruktion ist zum einen nachteilig, daß bei pertrochanterer Fraktur der Femurkopf gegenüber der Schenkelhalsschraube nicht gegen Verdrehen gesichert ist. Dabei ist zu bedenken, daß sich ein nicht unerheblicher Teil der Schenkelhalsschraube innerhalb der relativ weichen Spongiosa des Schenkelhalses erstreckt. Nur das selbstschneidende Gewinde reicht bis in die relativ feste Spongiosa des Femurkopfes. Bei starker Belastung ist jedoch eine Rotationsstabilität dieses Gewindes gegenüber dem Kopf-Halsfragment nicht sichergestellt, so daß es dann zu der erwähnten Verdrehung des Femurkopfes kommen kann.

Ganz ähnlich ist die Situation bei dem Osteosynthese-Hilfsmittel gemäß der EP 0 521 600 A1; auch dort ist der Schenkelhalsteil rotationssymmetrisch mit einen Gewindeabschnitt am freien Ende desselben ausgebildet. Des weiteren sind keine Vorkehrungen für eine axiale Festlegung des Schenkelhalsteils innerhalb der schrägen Durchgangsöffnung im Verriegelungsnagel vorgesehen. Bekannt ist jedoch aus dieser Druckschrift die schräge Durchgangsöffnung im proximalen Bereich des Verriegelungsnagels mit einem von einem kreis- bzw. rotationssymmetrischen Querschnitt abweichenden Querschnitt auszubilden. Der sich durch die schräge Durchgangsöffnung des Verriegelungsnagels hindurch erstreckende Abschnitt des Schenkelhalsteils weist einen komplementären Querschnitt auf. Durch diese Maßnahmen ist der Schenkelhalsteil rotationsstabil am Verriegelungsnagel befestigt. Die Rotationsstabilität des Schenkelhalses selbst gegenüber dem Schenkelhalsteil wird dadurch jedoch nicht gewährleistet im Hinblick darauf, daß der sich in die Spongiosa des Schenkelhalses hinein erstreckende Abschnitt des Schenkelhalsteils rotationssymmetrisch ausgebildet und am freien Ende mit einem rotationssymmetrischen Gewinde versehen ist. Schließlich fehlen auch Maßnahmen zur Erhöhung der Bruchfestigkeit des Verriegelungsnagels im Bereich der schrägen Durchgangsöffnung für den Schenkelhalsteil. Das gleiche gilt im übrigen für das Osteosynthese-Hilfsmittel gemäß der EP 0 321 170 A1.

Aus der US-A 4,103,683 ist es bekannt, den Schenkelhalsteil als Metallstreifen auszubilden, dessen femurkopfseitiges Ende eine spiralförmige Verwindung aufweist. Darüber hinaus erstreckt sich das so ausgebildete Schenkelhalsteil im rechten Winkel zum Verriegelungsnagel. Dazu ist zu sagen, daß der Schenkelhals relativ zum Femur in der Regel einen Winkel von etwa 125° einschließt. Dieser Winkel mag sich mit zunehmendem Alter verringern; auf jeden Fall ist es unwahrscheinlich, daß in der Natur der Schenkelhals einen Winkel von 90° zum Femur einschließt. Die Folge ist, daß bei Anwendung der bekannten Konstruktion nicht ausgeschlossen werden kann, daß nach einiger Zeit der Schenkelhalsteil aus dem Schenkelhals austritt. Darüber hinaus hat das spiralförmig verwundene Ende der Schenkelhalsteils bzw. der bekannten Schenkelhalsklinge zur Folge, daß diese durch einen entsprechenden Schlitz am proximalen Ende des Verriegelungsnagels hindurchgedreht werden muß. Dementsprechend verhält sich die bekannte Schenkelhalsklinge wie eine groß dimensionierte Schraube, die in den Schenkelhals eintritt und dort zu einer entsprechenden Beschädigung der Spongiosa führt. Auch wird durch die Verwendung der bekannten schenkelhalsklinge, die nach Art einer Schraube verwunden ist, der Femurkopf nicht gegen Verdrehung gesichert. Des weiteren verhält sich die bekannte Schenkelhalsklinge in Richtung etwa senkrecht zu der durch den Verriegelungsnagel einerseits und die Schenkelhalsklinge andererseits aufgespannten Ebene wie ein biegeelastisches Federblatt. Die Stabilität der bekannten Konstruktion in dieser Richtung ist entsprechend begrenzt.

Ausgehend von dem erwähnten Stand der Technik liegt der vorliegenden Erfindung die Aufgabe zugrunde, ein Osteosynthese-Hilfsmittel der aus der EP 0 321 170 A1 bekannten Art so weiterzubilden, daß auch bei stärkster Belastung eine hohe Bruchfestigkeit gewährleistet und eine Verdrehung des Femurkopfes vermieden ist.

Diese Aufgabe wird durch die kennzeichnenden Merkmale des Patentanspruches 1 gelöst.

Durch die erfindungsgemäße Ausbildung des Schenkelhalsteils als Schenkelhalsklinge wird diese zum einen rotationssymmetrisch im Verriegelungsnagel gehalten; zum anderen wird der Femurkopf durch die Schenkelhalsklinge gegen Verdrehen gesichert. Von besonderer Bedeutung für die Stabilität des erfindungsgemäßen Systems ist die beanspruchte Verdickung des Verriegelungsnagels im Bereich der schrägen Durchgangsöffnung für die Schenkelhalsklinge. Auf diese Weise wird selbst bei extrem hohen Belastungen ein Bruch des Verriegelungsnagels im Bereich dieser Durchgangsöffnung vermieden. Dabei ist zu bedenken, daß dieser Bereich stabilitätsmäßig äußerst kritisch ist, da er eine konstruktionsbedingt eine Schwachstelle darstellt, die darüber hinaus über die Schenkelhalsklinge relativ stark beansprucht ist. Durch die beanspruchte Verdickung wird eine ausreichende Bruchfestigkeit in diesem kritischen Bereich erreicht.

Der erfindungsgemäße Schenkelhalsteil ist schonend, d. h. unter minimaler Verdrängung von Spongiosa in den Schenkelhals einsetzbar. Der Schenkelhalsteil gemäß Erfindung zeichnet sich darüber hinaus durch eine hohe Biegesteifigkeit in sämtlichen Ebenen sowie hohe Verwindungssteifigkeit um seine Längsachse aus.

Vorteilhafte konstruktive Details der Erfindung sind in den Unteransprüchen beschrieben. Ergänzend dazu sei jedoch darauf hingewiesen, daß der Verriegelungsnagel in an sich bekannter Weise vorzugsweise durchgehend hohl ausgebildet ist, so daß er über einen Führungsdorn in den proximalen Femur einführbar ist. Die Wandstärke des hohlen Verriegelungsnagels beträgt etwa 1,5 bis 2,5, insbesondere etwa 2,0 mm.

Die Maßnahmen nach Anspruch 7, wonach in der durch Verriegelungsnagel und Schenkelhalsklinge definierten Ebene der proximale Abschnitt des Verriegelungsnagels gegenüber dessen distalem Abschnitt um etwa 4 bis 8°, insbesondere etwa 6°, lateral nach außen gebogen ist, wird eine minimale Spannungslinienkonzentration im Verriegelungsgleitnagel erhalten. Bei den bekannten Konstruktionen beträgt der vorgenannte Winkel etwa 12°. Diese deutliche Abknickung des Verriegelungsnagels führt zu Instabilitäten bei Wechseldruckbelastungen im Bereich der Abknickung, die durch den erfindungsgemäßen Kompromiß vermieden werden. Dabei soll auch darauf geachtet werden, daß der Übergang zwischen distalem und proximalem Abschnitt durch einen knickfreien Bogenabschnitt gebildet ist. Grundsätzlich wäre es natürlich vorteilhaft, den Verriegelungsnagel exakt geradlinig auszubilden. Dann würde die größte Knicksteifigkeit erreicht werden. Dies ist jedoch aus operationstechnischen Gründen nicht zweckmäßig; denn die Abwinkelung ist erforderlich, um eine Eintrittsstelle des Nagels im Bereich der Rollhügelspitze zu erreichen und nicht in den Bereich der Fossa trochanterica zu kommen. Letzteres wäre mit einer wesentlichen Beeinträchtigung der Durchblutung des bereits durch die Fraktur beeinträchtigten Kopf-Hals-Fragmentes verbunden. Des weiteren ist die Insertion eines Verriegelungsnagels im Bereich der Fossa trochanterica technisch sehr aufwendig, so daß sich die erfindungsgemäße Konstruktion gemäß Anspruch 7 als idealer Kompromiß zwischen technisch einfacher Insertion einerseits und Verminderung der Spannungslinienkonzentration im Verriegelungsnagel andererseits darstellt.

Die Länge des Verriegelungsnagels beträgt zwischen 150 und 350 mm, insbesondere etwa 220 mm zur Versorgung pertrochanterer Frakturen und etwa 320 mm zur Versorgung subtrochanterer und pathologischer Frakturen. Der etwas kürzere Verriegelungsnagel besitzt in einer Ansicht parallel zu der durch Verriegelungsnagel und Schenkelhalsklinge definierten Ebene einen geraden Verlauf. Damit kann der Verriegelungsnagel für den rechten und linken Femur in gleicher Weise ausgebildet werden. Bei der etwas längeren Ausführung muß die Krümmung des Femurs durch eine ventrale Konvexität mit einem Radius von etwa 1,5 m berücksichtigt werden. Dementsprechend ist dann ein Verriegelungsnagel für den rechten Femur und ein gesonderter Verriegelungsnagel für den linken Femur erforderlich.

In allen Fällen ist das distale Ende des Verriegelungsnagels vorzugsweise abgerundet, um ein sanftes Einführen in den Markraum des Femur zu gewährleisten.

Wie bereits oben erwähnt, ist von besonderer Bedeutung für die Stabilität des erfindungsgemäßen Systems die Maßnahme, daß die Breite des proximalen Abschnitts des Verriegelungsnagels im Bereich der schrägen Durchgangsöffnung für die Schenkelhalsklinge und in Richtung senkrecht zu dieser Durchgangsöffnung größer ist als die Breite bzw. der Außendurchmesser des relativ zu diesem Abschnitt distalen Bereichs des Verriegelungsnagels, wobei die Übergänge zwischen den Bereichen unterschiedlicher Breite jeweils knickfrei bzw. sanft gewölbt ausgebildet sein sollen, um Spannungsspitzen zu vermeiden. Auf diese Weise wird sicher ein Bruch des Verriegelungsnagels im Bereich der Durchgangsöffnung für die Schenkelhalsklinge vermieden. Dieser Bereich ist stabilitätsmäßig äußerst kritisch, da er eine Schwachstelle darstellt, die darüber hinaus über die Schenkelhalsklinge relativ stark beansprucht ist. Durch die vorgenannten Maßnahmen wird eine ausreichende Bruchfestigkeit in dem kritischen Bereich um die schräge Durchgangsöffnung für die Schenkelhalsklinge erreicht.

Am proximalen Ende des Verriegelungsnagels sind mindestens zwei, vorzugsweise drei gleichmäßig über den Umfang verteilt angeordnete Einschnitte zur formschlüssigen Verbindung mit komplementären Vorsprüngen an einem Zielgerät ausgebildet. Damit ist eine starre, insbesondere rotationsstabile Verbindung zwischen Zielgerät und Verriegelungsnagel möglich. Eine solche starre Verbindung ist für die Plazierung der Schenkelhalsklinge und der distalen Verriegelungselemente für den Verriegelungsnagel von großer Bedeutung. Damit wird eine exakte Handhabung gewährleistet.

Die Längsachse der schrägen Durchgangsöffnung für die Schenkelhalsklinge schließt mit der Längsachse des proximalen Abschnitts des Verriegelungsnagels einen Winkel von entweder 125° oder 135° ein. Diese beiden Ausführungsformen der Durchgangsöffnung im Verriegelungsnagel, die auch die Lage der Schenkelhalsklinge relativ zum Verriegelungsnagel definieren, haben sich in der Praxis als ausreichend herausgestellt. Mit diesen beiden Winkeln lassen sich alle physiologischen Variationen des Schenkelhals-Femurschaft-Winkels beherrschen. Eine ausgeprägte Valgisationsstellung um 140° und mehr ist bei dem erfindungsgemäßen System, das nicht von einer knöchernen kortikalen Kraftübertragung abhängig ist, nicht erforderlich. Dementsprechend sind bei Verwendung des erfindungsgemäßen Osteosynthese-Hilfsmittels auch nur zwei Zielbögen erforderlich, und zwar ein Zielbogen für 125° und ein weiterer Zielbogen für 135°. Man kommt also mit einer minimalen Anzahl von Instrumenten aus.

Der durchschnittliche Durchmesser des erfindungsgemäßen Verriegelungsnagels beträgt etwa 11 - 14 mm, vorzugsweise etwa 12 mm. Dieser relativ geringe Durchmesser reicht aus, da keine Kraftübertragung vom Verriegelungsnagel auf den Knochen stattfindet bzw. stattfinden soll. Die Krafteinleitung erfolgt über die Schenkelhalsklinge, den intramedullären Verriegelungsnagel auf die distalen Verriegelungselemente und von dort auf den distalen Femur. Ein großer rigider Verriegelungsnagel, der einen Knochenkontakt zum Femur herstellen würde, wäre nur von Nachteil. Es bestünde die Gefahr, daß der Femur durch den Nagel gesprengt wird, sowie erhöhte Frakturgefahr am Nagelende.

Sowohl in Kombination mit der vorbeschriebenen Konstruktion eines Osteosynthese-Hilfsmittels als auch unabhängig davon, d. h. für herkömmliche Konstruktionen mit Schenkelhalsschrauben sind die Maßnahmen nach Anspruch 14 von großer Bedeutung, wonach der Schenkelhalsteil, d. h. entweder Schenkelhalsklinge gemäß vorliegender Erfindung oder herkömmliche Schenkelhalsschraube an seinem bzw. ihrem äußeren, dem Femurkopf abgewandten Ende einen radial vorspringenden Absatz, insbesondere einen sich über den Umfang erstreckenden Kragen aufweist, der die laterale Einführung des Schenkelhalsteils in die schräge Durchgangsöffnung des Verriegelungsnagels begrenzt. Damit kann der Schenkelhalsteil nicht aus Versehen zu weit in die schräge Durchgangsöffnung des Verriegelungsnagels eingeschlagen werden mit der Gefahr, daß bei Belastung der Schenkelhalsteil in Varusposition abkippt. Es hat sich in der Praxis gezeigt, daß bei einem solchen Abkippen des Schenkelhalsteils dieser kaum noch auf normalem Wege entfernbar ist. Die vorgenannte Gefahr bei herkömmlichen Schenkelhalsschrauben besteht vor allem dann, wenn eine zu kurze Schenkelhalsschraube gewählt wird.

Gemäß Anspruch 15 ist das vordere bzw. im Femurkopf liegende Ende bzw. sind die vorderen Kanten des Profilabschnitts der Schenkelhalsklinge als Schneiden ausgebildet. Die vorgenannte Konstruktion der Schenkelhalsklinge zeichnet sich durch ein hohes Flächenträgheitsmoment und damit einen entsprechend großen Biegewiderstand bei vergleichsweise dünnwandiger Ausbildung aus. Es muß daher beim Einbringen bzw. Einschlagen der Schenkelhalsklinge in den Schenkelhals und Femurkopf nur wenig Spongiosa verdrängt werden. Die Einbringung der Schenkelhalsklinge ist vergleichsweise schonend, und zwar auch bedingt durch die Ausbildung der vorderen Kanten als Schneiden.

Schließlich ist noch von besonderer Bedeutung die Konstruktion gemäß Anspruch 16. Diese Konstruktion zeichnet sich dadurch aus, daß die mindestens eine Querbohrung im distalen Bereich des Verriegelungsnagels als Langloch-Querbohrung ausgebildet ist, durch die hindurch ein distales Verriegelungselement, insbesondere ein distaler Verriegelungsbolzen hindurchführbar und im Knochen verankerbar ist, wobei der Verriegelungsbolzen zur statischen distalen Verriegelung des Verriegelungsnagels am proximalen Ende und zur dynamischen distalen Verriegelung des Verriegelungsnagels am distalen Ende der Langloch-Querbohrung eingeführt wird. Dementsprechend weist die Langloch-Querbohrung entsprechend Anspruch 17 vorzugsweise eine Länge auf, die etwa dem doppelten Durchmesser des zugeordneten Verriegelungsbolzens entspricht. Grundsätzlich ist auch eine etwas kleinere oder größere axiale Erstreckung der Langloch-Querbohrung denkbar. Die vorgenannte Konstruktion erlaubt also sowohl eine statische distale Verriegelung des Verriegelungsnagels als auch dynamische distale Verriegelung des Verriegelungsnagels. Bei den bekannten Osteosynthese-Hilfsmitteln dieser Art ist dagegen nur eine statische distale Verriegelung des Verriegelungsnagels möglich, wobei die Verriegelung mittels einer durch eine distale Querbohrung hindurchgeführten Knochenschraube erfolgt. Erfindungsgemäß soll auch diese durch einen Verriegelungsbolzen ersetzt werden, der ein selbstschneidendes Gewinde mit nur minimaler Gewindetiefe aufweist, welches gerade ausreicht, um den Verriegelungsbolzen im Knochen zu halten. Ein Festziehen des Verriegelungsbolzens nach Art einer Schraube ist erfindungsgemäß nicht vorgesehen. Im Gegenteil, ein solches Festziehen soll erfindungsgemäß vermieden werden, da sich gezeigt hat, daß dadurch sehr häufig Schwachstellen für eine künftige Fraktur entstehen. Der Halt des erfindungsgemäßen Verriegelungsbolzens im Knochen soll gerade so groß sein, daß ein Verrutschen des Verriegelungsbolzens in Bezug zum Verriegelungsnagel intramedullär verhindert ist.

Der distale Verriegelungsbolzen gemäß Erfindung soll mittels eines Zielgeräts einbringbar sein, wobei dieses zur Plazierung des Verriegelungsbolzens in der Langloch-Querbohrung eine um 180° wendbare Zielbüchse mit einer Bohrung entsprechend dem Durchmesser des Verriegelungsbolzens aufweist. Konkret ist ein derartiges Zielgerät in Anspruch 19 beschrieben. Dabei reicht ein einziges Zielgerät sowohl für die rechte als auch die linke Seite des Patienten aus. Das Zielgerät wird am proximalen Ende des Verriegelungsnagels unter Ausbildung einer starren Verbindung mit diesem angeschlossen. Der Anschluß erfolgt entweder durch eine Schraub- oder Rastverbindung. Des weiteren soll darauf geachtet werden, daß das Zielgerät ohne lange Hebelarme auskommt, um ein Verformen, durch Anfassen an einem lateralen Griff, zu vermeiden. Des weiteren soll das Zielgerät einstückig ausgebildet sein, um Relativbewegungen zwischen Teilen des Zielgerätes zu vermeiden, die zu Abweichungen von vorbestimmten Zielen führen könnten (Position der Schenkelhalsklinge einerseits und Position der distalen Verriegelungselemente andererseits). Es muß also die Zielgenauigkeit des Systems gesichert sein.

Für die Positionierung der distalen Verriegelungselemente, insbesondere Verriegelungsbolzen, weist das erfindungsgemäße Zielgerät an einem im montierten Zustand sich etwa parallel zum Verriegelungsnagel und in Richtung zu dessen distalem Ende hin erstreckenden Arm mit mindestens einem der mindestens einen im distalen Bereich des Verriegelungsnagels ausgebildeten Langloch-Querbohrung zugeordnetes Langloch zur Aufnahme einer komplementären Zielbüchse auf, die an ihrem einen Ende eine Bohrung aufweist, deren Innendurchmesser geringfügig größer ist als der Außendurchmesser eines durch diese Bohrung hindurchzuführenden distalen Verriegelungsbolzens. Die Zielbüchse ist innerhalb des vorgenannten Langlochs um 180° wendbar, so daß die eine Bohrung in der Zielbüchse entweder dem proximalen oder dem distalen Ende der zugeordneten Langloch-Querbohrung im distalen Bereich des Verriegelungsnagels zustellbar ist. Die Zielbüchse wird nach Positionierung im vorgenannten Langloch des Zielgeräts mittels einer Feststellschraube oder dgl. verblockt.

Zum Einführen der erfindungsgemäßen Schenkelhalsklinge wird mittels des Zielgerätes der entsprechende Bereich im Schenkelhals und Femurkopf aufgebohrt, und zwar mittels eines Stufenbohrers, dessen vorderer Durchmesser etwa 3,5 mm und im übrigen etwa 10 mm beträgt, wobei der Bereich mit einem Durchmesser von 10 mm zum Aufbohren des Aufnahmeraums für den lateralen (distalen) Abschnitt der Schenkelhalsklinge dient. Für den medialen (proximalen) Profil-Abschnitt der Schenkelhalsklinge genügt eine Vorbohrung mit einem Durchmesser von etwa 3,5 mm. Die Aufbohrung erfolgt über den eingangs erwähnten Zieldraht für die Schenkelhalsklinge .

Zu den oben erwähnten Langloch-Querbohrungen im distalen Bereich des Verriegelungsnagels sei noch erwähnt, daß eine statische distale Verriegelung in der Regel bei normal verlaufenden A1 und A2 pertrochanteren oder Schenkelhalsfrakturen durchgeführt wird. Eine dynamische distale Verriegelung erfolgt z. B. bei einer Querfraktur vom Typ A3 oder subtrochanteren Fraktur.

Die obigen Ausführungen lassen schließlich noch erkennen, daß man bei Verwendung des erfindungsgemäßen Osteosynthese-Hilfsmittels eine minimale Anzahl von Instrumenten benötigt, nämlich:
- Zielgerät 125°;
- Zielgerät 135°;
- Bohr- und Zentrierhülse für die Schenkelhalsklinge;
- Bohr- und Zielbüchse für die distalen Verriegelungsbolzen;
- Zieldraht für die Schenkelhalsklinge (Durchmesser etwa 3,0 mm), mit zugehörigem Einschlaginstrument für die Schenkelhalsklinge, einem Schraubenzieher für die distalen Verriegelungsbolzen und für den der Schenkelhalsklinge zugeordneten Verriegelungsstift (Madenschraube).

Nachstehend wird eine Ausführungsform eines erfindungsgemäßen Osteosynthese-Hilfsmittels anhand der beigefügten Zeichnung näher erläutert. Es zeigen:
- Fig. 1: einen erfindungsgemäß ausgebildeten Verriegelungsnagel in Lateralansicht;
- Fig. 2: den Verriegelungsnagel gemäß Fig. 1 teilweise im Längsschnitt, teilweise in Ansicht;
- Fig. 3: eine Draufsicht auf das proximale Ende des Verriegelungsnagels in Richtung der Pfeile A-A in Fig. 2 und in vergrößertem Maßstab;
- Fig. 4: eine erfindungsgemäß ausgebildete und dem Verriegelungsnagel gemäß den Fig. 1 - 3 zugeordnete Schenkelhalsklinge in Seitenansicht;
- Fig. 5: das laterale (distale) Ende der Schenkelhalsklinge gemäß Fig. 4 in Draufsicht;
- Fig. 6: das mediale (proximale) Ende der Schenkelhalsklinge gemäß Fig. 4 in Draufsicht und in vergrößertem Maßstab;
- Fig. 7: den Profilabschnitt der Schenkelhalsklinge gemäß Fig. 4 im Schnitt längs Linie A-A in Fig. 4 und in vergrößertem Maßstab; und
- Fig. 8: die Zuordnung zwischen Verriegelungsnagel, Schenkelhalsklinge und zugeordnetem Zielgerät relativ zueinander und relativ zum Femur in schematischer Vorderansicht.

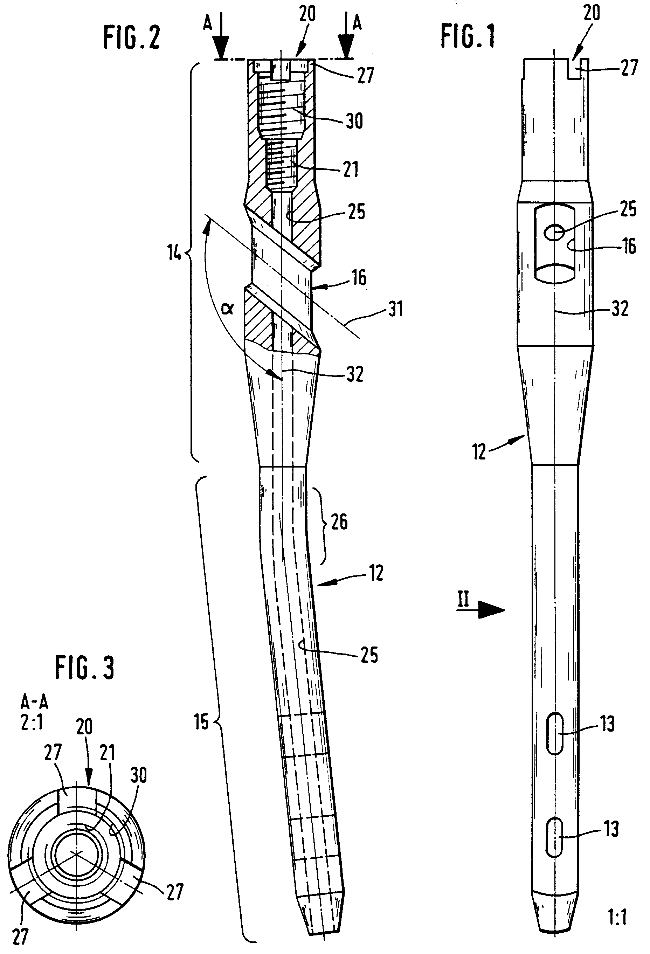

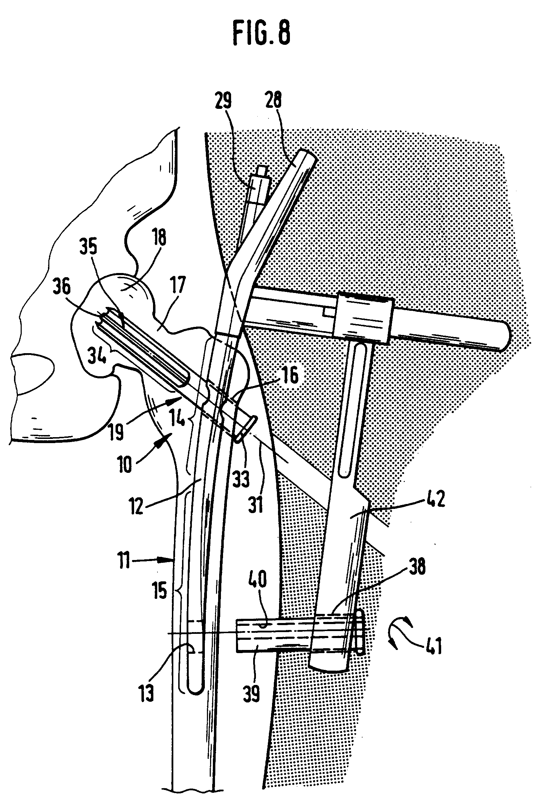

In den Fig. 1 - 8 ist ein Osteosynthese-Hilfsmittel 10 (siehe Fig. 8) zur Versorgung subtrochanterer und pertrochanterer sowie Schenkelhalsfrakturen mit einem von proximal in den Markraum eines Femur 11 einführbaren Verriegelungsnagel 12, der einen distalen Bereich mit zwei Langloch-Querbohrungen 13 für die Aufnahme eines distalen Verriegelungselements, nämlich nicht näher dargestellten Verriegelungsbolzens, sowie einen proximalen Abschnitt 14 mit einer schrägen Durchgangsöffnung 16 aufweist, und mit einem von lateral durch die schräge Durchgangsöffnung 16 in den Schenkelhals 17 und Femurkopf 18 einführbaren Schenkelhalsteil in Form einer Schenkelhalsklinge 19 mit rechteckförmigem Grundquerschnitt dargestellt. Der distale Bereich des Verriegelungsnagels 12 ist mit der Bezugsziffer 15 gekennzeichnet.

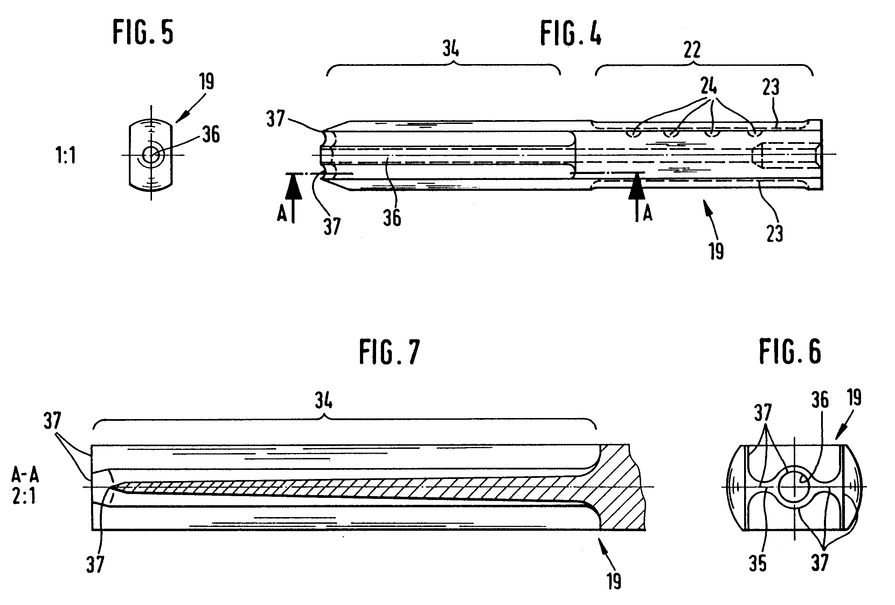

Entsprechend dem Querschnitt der Schenkelhalsklinge 19 ist der Querschnitt der schrägen Durchgangsöffnung 16 ausgebildet, wobei Schenkelhalsklinge 19 und Durchgangsöffnung 16 so relativ zueinander dimensioniert sind, daß die Schenkelhalsklinge 19 innerhalb der Durchgangsöffnung 16 spielfrei gehalten ist. In das proximale Ende 20 des Verriegelungsnagels 12 ist ein nicht näher dargestellter Verriegelungsstift (Madenschraube oder dgl.) einsetzbar, nämlich einschraubbar. Die entsprechende Gewindebohrung ist in Fig. 2 mit der Bezugsziffer 21 gekennzeichnet. Mittels des in die Gewindebohrung 21 einschraubbaren Verriegelungsstiftes kann die axiale Bewegung der Schenkelhalsklinge 19 innerhalb der schrägen Durchgangsöffnung 16 entweder begrenzt oder vollständig blockiert werden. Zu diesem Zweck weist der der Durchgangsöffnung 16 zugeordnete Abschnitt 22 (siehe Fig. 4) der Schenkelhalsklinge 19 zumindest an der proximalen (bei der hier dargestellten Ausführungsform auch an der distalen) Seite eine achsparallele Nut 23 auf, in die das in das proximale Ende 20 des Verriegelungsnagels 12 einsetzbare Verriegelungselement bzw. der vorerwähnte Verriegelungsstift in der beschriebenen Weise eingreift. Die eine Nut 23 kann zusätzlich im axialen Abstand voneinander angeordnete Vertiefungen 24 aufweisen, in die bei Bedarf der Verriegelungsstift eingreifen soll, um eine sichere starre Verbindung zwischen Verriegelungsnagel 12 und Schenkelhalsklinge 19 herstellen zu können. Damit soll bei Bedarf eine Axialbewegung der Schenkelhalsklinge 19 sicher vermieden werden. Je nach Bedarf wird bei der Schenkelhalsklinge 19 gemäß Fig. 4 entweder die Axialnut 23 mit den muldenförmigen Vertiefungen 24 oder die Axialnut 23 ohne die vorgenannten Vertiefungen dem Verriegelungsstift zugeordnet. Die erstgenannte Zuordnung wird insbesondere dann gewählt, wenn die Schenkelhalsklinge 19 innerhalb der schrägen Durchgangsöffnung 16 des Verriegelungsnagels 12 blockiert werden soll. Insofern ist die Ausführungsform gemäß Fig. 4 doppelfunktional.

Wie bereits erwähnt, ist der Schenkelhalsteil bei der hier beschriebenen Ausführungsform als Schenkelhalsklinge 19 mit einem von einem kreis- bzw. rotationssymmetrischen Querschnitt abweichenden Querschnitt, nämlich Rechteck-Querschnitt ausgebildet. Dadurch und durch den entsprechenden Querschnitt der schrägen Durchgangsöffnung 16 im Verriegelungsnagel 12 wird eine dauerhaft drehfeste Plazierung der Schenkelhalsklinge 19 innerhalb der schrägen Durchgangsöffnung 16 erreicht. Die rechteckförmige Durchgangsöffnung 16 ist so ausgebildet, daß die Schenkelhalsklinge 19 hochkant in der Durchgangsöffnung 16 plaziert ist. Die Flachseiten der Schenkelhalsklinge 19 erstrecken sich also parallel zur Längsachse des Verriegelungsnagels 12 bzw. Schenkelhalses. Die beschriebene Konstruktion führt zu einem besonders großen Biegewiderstand der Schenkelhalsklinge mit der Folge, daß keine Gefahr für ein Blockieren der Axialbewegung der Schenkelhalsklinge 19 bedingt durch eine Verbiegung derselben besteht. Bei dynamischer Plazierung der Schenkelhalsklinge 19 innerhalb der Durchgangsöffnung 16 bleibt die Dynamik sicher erhalten.

Wie der Fig. 2 entnommen werden kann, ist der Verriegelungsnagel 12 durchgehend hohl ausgebildet (Durchgangsbohrung 25), so daß er über einen nicht näher dargestellten, jedoch an sich bekannten Führungsspieß in den proximalen Femur einführbar ist. Die Wandstärke des hohlen Verriegelungsnagels 12 beträgt etwa 2,0 mm. Im übrigen ist der Verriegelungsnagel aus einem humanverträglichen Material, insbesondere Titan bzw. einer Titanlegierung hergestellt. Das gleiche gilt für die Schenkelhalsklinge 19 sowie alle anderen Teile, wie Schenkelhalsklingen-Verriegelungsstift und distale Verriegelungsbolzen.

Wie des weiteren der Fig. 2 und auch der Fig. 8 entnommen werden kann, ist in der durch Verriegelungsnagel 12 und Schenkelhalsklinge 19 definierten Ebene der proximale Abschnitt 14 des Verriegelungsnagels 12 gegenüber dessen distalem Abschnitt 15 um etwa 6° lateral nach außen gebogen, wobei der Übergang zwischen distalem und proximalem Abschnitt durch einen knickfreien Bogenabschnitt 26 gebildet ist.

Im übrigen weist der Verriegelungsnagel 12 bei der dargestellten Ausführungsform einen rotationssymmetrischen bzw. kreisrunden Querschnitt auf. Grundsätzlich ist auch ein ovaler Querschnitt denkbar, um die Rotationsstabilität des Osteosynthese-Hilfsmittels innerhalb des Femur zu fördern.

Zu dem nicht näher dargestellten Verriegelungsstift für die Schenkelhalsklingen 19 sei noch erwähnt, daß dieser in an sich bekannter Weise versenkt im proximalen Ende 20 des Verriegelungsnagels 12 angeordnet ist.

Um die Festigkeit des Verriegelungsnagels 12 im kritischen Bereich der Durchgangsöffnung 16 zu erhöhen, ist dieser Bereich im Verhältnis zum distalen Bereich 15 des Verriegelungsnagels 12 aufgeweitet. Konkret ist der Außendurchmesser des Verriegelungsnagels 12 im Bereich der Durchgangsöffnung 16 etwa doppelt so groß wie der Durchmesser des distalen Bereichs 15. Damit ist gewährleistet, daß auch im Bereich der Durchgangsöffnung 16 die oben genannte Wandstärke des Verriegelungsnagels 12 erhalten wird. Die Gefahr eines Bruchs des Verriegelungsnagels 12 im Bereich der Durchgangsöffnung 16 wird auf diese Weise ausgeschlossen.

Im übrigen sollen sämtliche Übergänge zwischen distalem Bereich 15 und verbreitertem proximalen Abschnitt 14 knickfrei bzw. sanft gewölbt ausgebildet sein, um Spannungsspitzen in den genannten Übergangsbereichen zu vermeiden.

Entsprechend den Fig. 1 - 3, insbesondere entsprechend Fig. 3 sind am proximalen Ende 20 des Verriegelungsnagels 12 drei gleichmäßig über den Umfang verteilt angeordnete Einschnitte 27 zur formschlüssigen Verbindung mit komplementären Vorsprüngen an einem Zielgerät ausgebildet, so wie es in Fig. 8 mit der Bezugsziffer 28 angedeutet ist. Durch diese formschlüssige Verbindung, die zusätzlich kombiniert wird mit einer kraftschlüssigen Schraubverbindung (Schraubbolzen 29 in Fig. 8 und zugehörige Gewindebohrung 30 im proximalen Ende 20 des Verriegelungsnagels 12 entsprechend Fig. 2), wird eine starre Verbindung zwischen Zielgerät 28 und Verriegelungsnagel 12 erhalten, die erforderlich ist, um die Schenkelhalsklinge 19 sowie distalen Verriegelungsbolzen zielgenau positionieren zu können.

Die Längsachse 31 der schrägen Durchgangsöffnung 16 für die Schenkelhalsklinge 19 schließt mit der Längsachse 32 des proximalen Abschnitts 14 des Verriegelungsnagels 12 einen Winkel α von entweder 125° oder alternativ 135° ein. Hinsichtlich der Auswahl dieser beiden Alternativen wird auf die obigen Ausführungen hingewiesen.

Die Schenkelhalsklinge 19 weist entsprechend Fig. 8 an ihrem äußeren, dem Femurkopf 18 abgewandten Ende einen radial vorspringenden Absatz in Form eines sich über den Umfang erstreckenden Kragens 33 auf, der die laterale Einführung der Schenkelhalsklinge 19 in die schräge Durchgangsöffnung 16 des Verriegelungsnagels 12 begrenzt. Dieser radial vorspringende Absatz bzw. Kragen 33 kann auch mit Erfolg bei herkömmlichen Schenkelhalsschrauben ausgeführt sein, um die eingangs genannten Gefahren beim Einschlagen der Schenkelhalsklinge bzw. Schenkelhalsschraube zu vermeiden. Insofern handelt es sich um ein gesondertes und für die Praxis bedeutendes Konstruktionsmerkmal.

Entsprechend den Fig. 4, 6, 7 und 8 ist der im Femurkopf 18 und Schenkelhals 17 verankerbare Abschnitt 34 der Schenkelhalsklinge 19 als Doppel-T bzw. I-Profil 35 mit einer zentralen Durchgangsbohrung 36 für einen nicht näher dargestellten, jedoch an sich bekannten Führungsdraht ausgebildet. Die vorderen im Femurkopf 18 liegenden Kanten des Profilabschnitts 34 sind als meißelartige Schneiden 37 ausgebildet, um das Einschlagen der Schenkelhalsklinge 19 in die Spongiosa des Schenkelhalses und Femurkopfes ohne vorheriges Aufbohren der Spongiosa auf volle Breite der Schenkelhalsklinge zu erleichtern. Wie bereits eingangs erwähnt, wird mit der beschriebenen Ausbildung der Schenkelhalsklinge 19 mit einem Doppel-T bzw. I-Profilabschnitt 34 ein extrem hohes Flächenträgheitsmoment und damit extrem hohe Biegesteifigkeit bei minimaler Querschnittsfläche erreicht, so daß beim Einschlagen der Schenkelhalsklinge in den Schenkelhals und Femurkopf nur wenig Spongiosa verdrängt werden muß. Darüber hinaus begünstigt der Profilabschnitt 34 die Rotationsstabilität des Femurkopfes und Schenkelhalses im Verhältnis zum Femur bei Versorgung eines Schenkelhalsbruches oder einer pertrochanteren Fraktur.

Die beiden distalen Querbohrungen 13 im distalen Bereich des Verriegelungsnagels 12 sind als Langloch-Querbohrungen ausgebildet, durch die hindurch distale Verriegelungselemente, nämlich Verriegelungsbolzen zur Verankerung im Femurknochen durchführbar sind, wobei die Verriegelungsbolzen zur statischen distalen Verriegelung des Verriegelungsnagels 12 am proximalen Ende (in Fig. 1 und 2 oberen Ende) und zur dynamischen distalen Verriegelung des Verriegelungsnagels 12 am distalen Ende (in Fig. 1 und 2 unteren Ende) der Langloch-Querbohrungen 13 eingeführt werden. Bei der dargestellten Ausführungsform weisen die Langloch-Querbohrungen 13 jeweils eine axiale Erstreckung bzw. Länge auf, die etwa dem 2,5fachen Durchmesser der zugeordneten Verriegelungsbolzen bzw. der Breite der Langloch-Querbohrungen 13 entspricht.

Die distalen Verriegelungsbolzen werden mittels eines Zielgeräts 28 eingebracht. Wie bereits oben ausgeführt, sollen die distalen Verriegelungsbolzen an ihren distalen Enden jeweils ein selbstschneidendes Gewinde mit minimaler Gewindetiefe aufweisen. Das Gewinde soll gerade ausreichen, um die Verriegelungsbolzen im Femurknochen zu halten. Eine Klemm-Verschraubung soll zur Schonung des Knochens im Gegensatz zum Stand der Technik nicht möglich sein.

Anhand der Fig. 8 sei noch auf ein für das beschriebene Osteosynthese-Hilfsmittel geeignetes Zielgerät mit der Bezugsziffer 28 hingewiesen. Dieses ist am proximalen Ende 20 des Verriegelungsnagels 12 unter Ausbildung einer starren Verbindung mit diesem anschließbar. Es weist an einem im montierten Zustand sich etwa parallel zum Verriegelungsnagel 12 und in Richtung zu dessen distalem Ende hin mindestens ein der mindestens einen im distalen Bereich des Verriegelungsnagels 12 ausgebildeten Langloch-Querbohrung 13 zugeordnetes Langloch 38 zur Aufnahme einer komplementären Zielbüchse 39 auf, die an ihrem einen Ende eine Bohrung 40 umfaßt, deren Innendurchmesser geringfügig größer ist als der Außendurchmesser eines durch diese Bohrung hindurchzuführenden distalen Verriegelungsbolzens. Die Zielbüchse 39 ist im Langloch 38 um 180° wendbar, so daß die Bohrung 40 entweder dem proximalen oder dem distalen Ende der zugeordneten Langloch-Querbohrung 13 im distalen Bereich des Verriegelungsnagels 12 zustellbar ist. Die Möglichkeit, die Zielbüchse 39 in der beschriebenen Weise um 180° zu wenden, ist in Fig. 8 mit dem Doppelpfeil 41 angedeutet. Die Bohrung 40 dient zugleich als Bohrhülse für einen Bohrer, mit dem die Aufnahmeöffnungen im Femur für die distalen Verriegelungsbolzen vorgebohrt werden. In Verlängerung der Schenkelhalsklinge 19 weist der vorgenannte Arm 42 einen Durchgang zur Aufnahme einer Bohr- und Zentrierhülse für die Schenkelhalsklinge auf. Diese beiden Hülsen sind in Fig. 8 nicht näher dargestellt, da es sich diesbezüglich um an sich bekannte Konstruktionselemente handelt. Im übrigen ist das Zielgerät 28 einstückig ausgebildet, um Relativverschiebungen von einzelnen Bauteilen des Zielgerätes zu vermeiden. Dadurch wird eine hohe Zielgenauigkeit für das Einbringen der Schenkelhalsklinge sowie der distalen Verriegelungsbolzen nach Anschluß am Verriegelungsnagel 12 erreicht.

Werden sämtliche Konstruktionsmerkmale des beschriebenen Systems vereinigt, erhält man im Vergleich zum Stand der Technik ein optimales Osteosynthese-Hilfsmittel, wie erste Versuche des Erfinders bereits gezeigt haben.

Sämtliche in den Anmeldungsunterlagen offenbarten Merkmale werden als erfindungswesentlich beansprucht, soweit sie einzeln oder in Kombination gegenüber dem Stand der Technik neu sind.

## Patentansprüche

1. Osteosynthese-Hilfsmittel (10) mit einem Verriegelungsnagel (12), einem Schenkelhalsteil (19) und einem diesem zugeordneten Verriegelungsstift, zur Versorgung subtrochanterer, pertrochanterer und Schenkelhalsfrakturen mit einem von proximal in den Markraum eines Femur (11) einführbaren Verriegelungsnagel (12), der einen distalen Bereich (15) mit mindestens einer Querbohrung (13) für die Aufnahme eines distalen Verriegelungselements, sowie einen proximalen Abschnitt (14) mit einer schrägen Durchgangsöffnung (16) aufweist, und mit einem von lateral durch die schräge Durchgangsöffnung (16) hindurch in den Schenkelhals (17) und Femurkopf (18) einführbaren Schenkelhalsteil (19), wobei der Verriegelungsstift in das proximale Ende (20) des Verriegelungsnagels (12) einsetzbar ist so, daß mit diesem die axiale Bewegung des Schenkelhalsteils (19) innerhalb der schrägen Durchgangsöffnung (16) im proximalen Abschnitt (14) des Verriegelungsnagels (12) entweder begrenzbar oder vollständig blockierbar ist,
**dadurch gekennzeichnet,** daß
der Schenkelhalsteil als Schenkelhalsklinge (19) mit einem von einem kreis- bzw. rotationssymmetrischen Querschnitt abweichenden Querschnitt, insbesondere Rechteck-Querschnitt, ausgebildet ist, wobei die schräge Durchgangsöffnung (16) für die Aufnahme der Schenkelhalsklinge (19) einen komplementären, d. h. ebenfalls insbesondere Rechteck-Querschnitt aufweist, derart, daß die Schenkelhalsklinge (19) dauerhaft spielfrei und rotationsstabil innerhalb der schrägen Durchgangsöffnung (16) gehalten ist, daß zumindest der im Femurkopf (18) verankerbare Abschnitt (34) der Schenkelhalsklinge (19) als Doppel-T bzw. I- (35), T-, Stern-, U- oder dgl. -Profil ausgebildet ist, und daß die Breite des Verriegelungsnagels (12) im Bereich der schrägen Durchgangsöffnung (16) in Richtung senkrecht zu dieser größer ist als die Breite bzw. der Außendurchmesser im benachbarten Bereich des Verriegelungsnagels (12).

2. Hilfsmittel nach Anspruch 1,
**dadurch gekennzeichnet,** daß
der Schenkelhalsteil noch eine zentrale Durchgangsbohrung (36) für einen Führungsdraht umfaßt.

3. Hilfsmittel nach Anspruch 1 oder 2,
**dadurch gekennzeichnet,** daß
im Bereich der schrägen Durchgangsöffnung (16) an der oberen bzw. lateral/cranialen, ggf. auch unteren bzw. medial/caudalen Seite der Schenkelhalsklinge (19) eine achsparallele Nut (23) ausgebildet ist, in die der in das proximale Ende (20) des Verriegelungsnagels (12) einsetzbare Verriegelungsstift oder dgl. eingreift.

4. Hilfsmittel nach einem der Ansprüche 1 bis 3,
**dadurch gekennzeichnet,** daß
bei Verwendung einer Schenkelhalsklinge (19) mit rechteckförmigem Querschnitt diese hochkant in der schrägen Durchgangsöffnung (16) plaziert ist.

5. Hilfsmittel nach einem der Ansprüche 1 bis 4,
**dadurch gekennzeichnet,** daß
der Verriegelungsnagel (12) durchgehend hohl ausgebildet ist, so daß er über einen Führungsspieß in den proximalen Femur einführbar ist.

6. Hilfsmittel nach Anspruch 5,
**dadurch gekennzeichnet,** daß
die Wandstärke des hohlen Verriegelungsnagels (12) etwa 1,5 - 2,5 mm, insbesondere etwa 2,0 mm beträgt.

7. Hilfsmittel nach einem der Ansprüche 1 - 6,
**dadurch gekennzeichnet,** daß
in der durch Verriegelungsnagel (12) und Schenkelhalsklinge (19) definierten Ebene der proximale Abschnitt (14) des Verriegelungsnagels (12) gegenüber dessen distalem Abschnitt (15) um etwa 4 - 8°, insbesondere etwa 6° lateral nach außen gebogen ist, wobei der Übergang zwischen distalem und proximalem Abschnitt durch einen knickfreien Bogenabschnitt (26) gebildet ist.

8. Hilfsmittel nach einem der Ansprüche 1 - 7,
**dadurch gekennzeichnet,** daß
der Verriegelungsnagel (12) einen rotationssymmetrischen bzw. kreisrunden oder ovalen Querschnitt aufweist.

9. Hilfsmittel nach einem der Ansprüche 1 - 8,
**dadurch gekennzeichnet,** daß
die Länge des Verriegelungsnagels (12) zwischen 150 und 350 mm, insbesondere etwa 220 mm zur Versorgung von Schenkelhals- und pertrochanteren Frakturen und etwa 320 mm zur Versorgung subtrochanterer oder pathologischer Frakturen beträgt.

10. Hilfsmittel nach einem der Ansprüche 1 - 9,
**dadurch gekennzeichnet,** daß
der der Schenkelhalsklinge (19) zugeordnete Verriegelungsstift oder dgl. versenkt im proximalen Ende (20) des Verriegelungsnagels (12) angeordnet ist.

11. Hilfsmittel nach einem der Ansprüche 5 - 10,
dadurch gekennzeichnet, daß bei einem hohlen Verriegelungsnagel (12) die Nagel-Wandstärke im Bereich der schrägen Durchgangsöffnung (16) mindestens so groß ist wie die Wandstärke des Verriegelungsnagels (12) im distalen Bereich.

12. Hilfsmittel nach einem der Ansprüche 1 - 11,
**dadurch gekennzeichnet,** daß
am proximalen Ende (20) des Verriegelungsnagels (12) mindestens zwei, insbesondere drei gleichmäßig über den Umfang verteilt angeordnete Einschnitte (27) zur formschlüssigen Verbindung mit komplementären Vorsprüngen an einem Zielgerät (28) ausgebildet sind.

13. Hilfsmittel nach einem der Ansprüche 1 - 12,
**dadurch gekennzeichnet,** daß
die Längsachse (31) der schrägen Durchgangsöffnung (16) für die Schenkelhalsklinge (19) mit der Langsachse (32) des proximalen Abschnitts (14) des Verriegelungsnagels (12) einen Winkel (α) von entweder 125° oder alternativ 135° einschließt.

14. Osteosynthese-Hilfsmittel nach einem oder mehreren der vorhergehenden Ansprüche,
**dadurch gekennzeichnet,** daß
der Schenkelhalsteil (19) an seinem äußeren, dem Femurkopf (18) abgewandten Ende einen radial vorspringenden Absatz, insbesondere einen sich über den Umfang erstreckenden Kragen (33) aufweist, der die laterale Einführung des Schenkelhalsteils (19) in die schräge Durchgangsöffnung (16) des Verriegelungsnagels (12) begrenzt.

15. Hilfsmittel nach Anspruch 14,
**dadurch gekennzeichnet,** daß
das vordere bzw. im Femurkopf (18) liegende Ende bzw. die vorderen Kanten des Profilabschnitts (34) des Schenkelhalsteils, insbesondere der Schenkelhalsklinge (19) als Schneide bzw. Schneiden (37) ausgebildet ist bzw. sind.

16. Osteosynthese-Hilfsmittel nach einem oder mehreren der vorhergehenden Ansprüche,
**dadurch gekennzeichnet,** daß
die mindestens eine Querbohrung im distalen Bereich (15) des Verriegelungsnagels (12) als Langloch-Querbohrung (13) ausgebildet ist, durch die hindurch ein distales Verriegelungselement, insbesondere ein distaler Verriegelungsbolzen zur Verankerung im Femurknochen hindurchführbar ist, wobei der Verriegelungsbolzen zur statischen distalen Verriegelung des Verriegelungsnagels (12) am proximalen Ende und zur dynamischen distalen Verriegelung des Verriegelungsnagels (12) am distalen Ende der Langloch-Querbohrung (13) eingeführt ist, wobei der distale Verriegelungsbolzen ein selbstschneidendes Gewinde mit minimaler Gewindetiefe aufweist, welches gerade noch ausreicht, um den Verrieglungsbolzen im Femurknochen zu halten.

17. Hilfsmittel nach Anspruch 16,
**dadurch gekennzeichnet,** daß
die Langloch-Querbohrung (13) eine Länge aufweist, die etwa dem doppelten Durchmesser des zugeordneten Verriegelungsbolzens bzw. der Breite der Langloch-Querbohrung entspricht.

18. Hilfsmittel nach Anspruch 16 oder 17,
**dadurch gekennzeichnet,** daß
der distale Verriegelungsbolzen mittels eines Zielgeräts (28) einbringbar ist, wobei dieses zur Plazierung des Verriegelungsbolzens in der Langloch-Querbohrung (13) eine um 180° wendbare Zielbüchse (39) mit einer Bohrung (40) entsprechend dem Durchmesser des Verriegelungsbolzens aufweist.

19. Osteosynthese-Hilfsmittel (10) nach einem oder mehreren der vorangehenden Ansprüche, umfassend ein Zielgerät mit Zielbüchse (39),
**dadurch gekennzeichnet,** daß
das Zielgerät am proximalen Ende (20) des Verriegelungsnagels (12) unter Ausbildung einer starren Verbindung mit diesem form- und kraftschlüssig anschließbar ist, sowie an einem im montierten Zustand sich etwa parallel zum Verriegelungsnagel (12) und in Richtung zu dessen distalem Ende hin erstreckenden Arm (42) mindestens ein der mindestens einen im distalen Bereich des Verriegelungsnagels (12) ausgebildeten Langloch-Querbohrung (13) zugeordnetes Langloch (38) zur Aufnahme einer komplementären Zielbüchse (39) aufweist, die an ihrem einen Ende eine Zielbohrung (40) aufweist, deren Innendurchmesser geringfügig größer ist als der Außendurchmesser eines durch diese Bohrung hindurchzuführenden Knochenbohrers und/oder distalen Verriegelungsbolzens, und die um 180° wendbar ist, so daß die eine Zielbohrung (40) entweder dem proximalen oder dem distalen Ende der zugeordneten Langloch-Querbohrung (13) im distalen Bereich (15) des Verriegelungsnagels (12) zustellbar ist.

## Claims

1. Osteosynthetic aid (10) with a locking nail (12), with a neck-of-femur portion (19) and associated locking pin, for treatment of sub-trochantic, pertrochantic fractures and fractures of the neck of the femur with a locking nail (12) introducible from the proximal direction into the marrow space of a femur (11), said nail (12) having a distal area (15) with at least one transverse bore (13) for receiving a distal locking member, and a proximal section (14) with an oblique passage opening (16), and with a neck-of-femur portion (19) introducible laterally through the oblique passage opening (16) into the neck of the femur (17) and the head of the femur (18), the locking pin being insertable into the proximal end (20) of the locking nail (12) in such a way that thereby axial movement of the neck-of-femur portion (19) within the oblique passage opening (16) in the proximal section (14) of the locking nail (12) may either be limited or entirely suppressed,
characterised in that
the neck-of-femur portion is designed as a neck-of-femur blade (19) with a cross-section, particularly a rectangular cross-section, deviating from a circularly or rotarily symmetrical cross-section, the oblique passage opening (16), in order to receive the neck-of-femur blade (19) having a complementary, i.e. likewise in particular rectangular cross-section, in such a way that the neck-of-femur blade (19) is permanently held without play and in a rotarily stable manner within the oblique passage opening (16), in that at least the section (34) of the neck-of-femur blade (19) anchorable in the head of the femur (18) is designed as a double-T- or I- (35), T-, Star-, U- or the like -shaped profile, and in that the width of the locking nail (12) in the area of the oblique passage opening (16) is greater in a perpendicular direction thereto than the width or outer diameter in the adjacent area of the locking nail (12).

2. Aid according to claim 1,
characterised in that
the neck-of-femur portion also comprises a central passage bore (36) for a guide wire.

3. Aid according to claim 1 or 2,
characterised in that
in the area of the oblique passage opening (16) on the upper or lateral oblique cranial, if necessary also the lower or medial/caudal side of the neck-of-femur blade (19) there is formed an axially parallel groove (23) into which the locking pin or the like insertable into the proximal end (20) of the locking nail (12) engages.

4. Aid according to one of claims 1 to 3,
characterised in that
when a neck-of-femur blade (19) with a rectangular cross-section is used, the latter is placed on edge in the oblique passage opening (16).

5. Aid according to one of claims 1 to 4,
characterised in that
the locking nail (12) is continuously hollow in shape, so that it is introducible via a guide spike into the proximal femur.

6. Aid according to claim 5,
characterised in that
the wall thickness of the hollow locking nail (12) comes to about 1.5 - 2.5 mm, particularly about 2.0 mm.

7. Aid according to one of claims 1 to 6,
characterised in that
in the plane defined by the locking nail (12) and the neck-of-femur blade (19) the proximal section (14) of the locking nail (12) is bent laterally outwards with respect to its distal section (15) by about 4 to 8°, particularly about 6°, the transition between distal and proximal section being formed by a smooth arcuate section (26).

8. Aid according to one of claims 1 to 7,
characterised in that
the locking nail (12) has a rotarily symmetrical, or circular, or oval cross-section.

9. Aid according to one of claims 1 to 8,
characterised in that
the length of the locking nail (12) comes to between 150 and 350 mm, particularly about 220 mm, for the treatment of fractures of the neck of the femur and pertrochantic fractures, and about 320 mm for the treatment of subtrochantic or pathological fractures.

10. Aid according to one of claims 1 to 9,
characterised in that
the locking pin or the like associated with the neck-of-femur blade (19) is countersunk in the proximal end (20) of the locking nail (12).

11. Aid according to one of claims 5 to 10,
characterised in that
when the locking nail (12) is hollow, the wall thickness of the nail in the region of the oblique passage opening (16) is at least as great as the wall thickness of the locking nail (12) in the distal region.

12. Aid according to one of claims 1 to 11,
characterised in that
there are provided at the proximal end (20) of the locking nail (12) at least two, particularly three incisions (27), uniformly distributed over the circumference, for positive connection with complementary projections on an aiming device (28).

13. Aid according to one of claims 1 to 12,
characterised in that
the longitudinal axis (31) of the oblique passage opening (16) for the neck-of-femur blade (19) encloses with the longitudinal axis (32) of the proximal section (14) of the locking nail (12) an angle (α) of either 125° or alternatively 135°.

14. Osteosynthetic aid according to one or more of the preceding claims,
characterised in that
the neck-of-femur portion (19) has at its outer end facing away from the head of the femur (18) a radially projecting projection, in particular a collar (33) extending over the circumference, which limits the lateral introduction of the neck-of-femur portion (19) into the oblique passage opening (16) of the locking nail (12).

15. Aid according to claim 14,
characterised in that
the forward end, or the end lying in the head of the femur (18), or the forward edges of the profiled section (34) of the neck-of-femur portion, particularly of the neck-of-femur blade (19), is or are designed as a cutting edge or cutting edges (37).

16. Osteosynthetic aid according to one or more of the preceding claims,
characterised in that
the at least one transverse bore in the distal area (15) of the locking nail (12) is designed as a slotted transverse bore (13) through which a distal locking member, in particular a distal locking bolt is introducible in order to provide anchorage in the femur bone, said locking bolt being introducible, for static distal locking of the locking nail (12) at the proximal end, and for dynamic distal locking of the locking nail (12) at the distal end of the slotted transverse bore (13,) the distal locking bolt having a self-tapping thread with a minimum thread pitch, which is just sufficient to hold the locking bolt in the femur bone.

17. Aid according to claim 16,
characterised in that
the slotted transverse bore (13) has a length which corresponds to roughly twice the diameter of the associated locking bolt, or to the width of the slotted transverse bore.

18. Aid according to claim 16 or 17,
characterised in that
the distal locking bolt is introducible by means of an aiming device (28), this latter, in order to place the locking bolt in the slotted transverse bore (13), having an aiming tube (39) rotatable through 180°, with a bore (40) corresponding to the diameter of the locking bolt.

19. Osteosynthetic aid (10) according to one or more of the preceding claims, comprising an aiming device with aiming tube (39),
characterised in that
the aiming device may be positively and frictionally connected at the proximal end (20) of the locking nail (12) forming a rigid connection therewith, and, on an arm (42) extending in the assembled condition roughly parallel to the locking nail (12) and extending in the direction of its distal end, at least one slot (38) associated with the at least one slotted transverse bore (13) formed in the distal region of the locking nail (12), for receiving a complementary aiming tube (39) which has at one end an aiming bore (40) whose inner diameter is slightly greater than the outer diameter of a bone drill and/or distal locking bolt to be passed through this bore, and which is rotatable through 180°, so that the one aiming bore (40) may be associated either with the proximal or with the distal end of the associated slotted transverse bore (13) in the distal region (15) of the locking nail (12).

## Revendications

1. Instrument auxiliaire pour ostéosynthèse (10) avec une aiguille de fixation (12), une pièce pour col de fémur (19) et une fiche de fixation associée à ceux-ci permettant de traiter des fractures subtrochanterienne et pertochanterienne, ainsi que des fractures du col de fémur, comprenant une aiguille de fixation (12) susceptible d'être insérée du côté proximal dans l'espace de la moelle d'un fémur (11), laquelle aiguille présente une zone distale (15) avec au moins un perçage transversal (13) pour recevoir un élément de fixation distal ainsi qu'une section proximale (14) avec une ouverture traversante (16) oblique ; comprenant une pièce pour col de fémur (19) susceptible d'être insérée en partant du côté au travers de l'ouverture traversante oblique (16) jusque dans le col de fémur (17) et dans la tête de fémur (18) ; dans lequel la fiche de fixation peut être installée dans l'extrémité proximale (20) de l'aiguille de fixation (12) de telle sorte que, par cette fiche, le déplacement axial de la pièce pour col de fémur (19) peut être soit limité soit totalement bloqué à l'intérieur de l'ouverture traversante oblique (16) en la section proximale (14) de l'aiguille de fixation (12), caractérisé en ce que la pièce pour col de fémur est réalisée sous la forme d'une lame (19) pour col de fémur ayant une section décroissante à partir d'une section circulaire, respectivement symétrique en rotation, en une section en particulier à angle droit, pour laquelle l'ouverture traversante oblique (16) recevant cette lame pour col de fémur (19) présente une section complémentaire, c'est-à-dire également en particulier à angle droit, de telle sorte que cette lame pour col de fémur (19) est retenue de manière durable sans jeu et stable en rotation à l'intérieur de l'ouverture traversante oblique (16) ; en ce qu'au moins le tronçon (34) de cette lame pour col de fémur (19) ancré dans la tête de fémur (18) est réalisé avec un profil en double-T, respectivement en I- (35), T-, en étoile, en U ou analogue, et en ce que la largeur de l'aiguille de fixation (12) dans la zone de l'ouverture traversante oblique (16), cette largeur étant vue perpendiculairement à cette ouverture, soit plus grande que la largeur, respectivement le diamètre extérieur, des zones adjacentes de l'aiguille de fixation (12).

2. Instrument auxiliaire selon la revendication 1, caractérisé en ce que la pièce de col de fémur comprend en outre un perçage traversant central (36) pour un fil de guidage.

3. Instrument auxiliaire selon la revendication 1 ou 2, caractérisé en ce qu'une rainure (23) parallèle à l'axe est réalisée en le côté supérieur, respectivement latéral/cranial, le cas échéant aussi en le côté inférieur, respectivement médial/caudal, de la lame pour col de fémur (19) dans la zone correspondante de l'ouverture traversante oblique (16), rainure dans laquelle accroche la fiche de fixation susceptible d'être installée dans l'extrémité proximale (20) de l'aiguille de fixation (12).

4. Instrument auxiliaire selon l'une des revendications 1 à 3, caractérisé en ce que lors de l'utilisation d'une lame pour col de fémur (19) à section à angle droit, celle-ci est placée sur le champ dans l'ouverture traversante oblique (16).

5. Instrument auxiliaire selon l'une des revendications 1 à 4, caractérisé en ce que l'aiguille de fixation (12) est réalisée de manière creuse traversante de telle sorte qu'elle puisse être engagée dans la partie proximale du fémur par dessus une broche de guidage.

6. Instrument auxiliaire selon la revendication 5, caractérisé en ce que l'épaisseur de paroi de l'aiguille creuse de fixation (12) est de l'ordre de 1,5 - 2,5 mm, en particulier de l'ordre de 2,0 mm.

7. Instrument auxiliaire selon l'une des revendications 1 - 6, caractérisé en ce que, dans le plan défini par l'aiguille de fixation (12) et la lame pour col de fémur (19), la section proximale (14) de l'aiguille de fixation (12) est courbée latéralement vers l'extérieur par rapport à sa zone distale (15) d'environ 4 - 8 degrés, en particulier 6 degrés, forme dans laquelle la transition entre la zone distale et proximale est réalisée sous la forme d'une zone courbe (26) sans pliure.

8. Instrument auxiliaire selon l'une des revendications 1 - 7, caractérisé en ce que l'aiguille de fixation (12) présente une section symétrique en rotation, respectivement circulaire ou ovale.

9. Instrument auxiliaire selon l'une des revendications 1 - 8, caractérisé en ce que la longueur de l'aiguille de fixation (12) est comprise entre 150 mm et 350 mm, en particulier de l'ordre de 220 mm, pour le traitement d'une fracture du col de fémur ou pertochanterienne, et de l'ordre de 320 mm pour le traitement d'une fracture subtrochanterienne ou pathologique.

10. Instrument auxiliaire selon l'une des revendications 1 - 9, caractérisé en ce que la fiche de fixation ou analogue associée à la lame pour col de fémur (19) est agencée de manière entièrement effacée dans l'extrémité proximale (20) de l'aiguille de fixation (12).

11. Instrument auxiliaire selon l'une des revendications 5 - 10, caractérisé en ce que, pour une aiguille creuse de fixation (12), l'épaisseur de paroi d'aiguille dans la zone de l'ouverture traversante oblique (16) est au moins aussi grande que l'épaisseur de paroi d'aiguille de fixation (12) dans la zone distale.

12. Instrument auxiliaire selon l'une des revendications 1 - 11, caractérisé en ce que dans l'extrémité proximale (20) de l'aiguille de fixation (12) sont ménagées au moins deux, en particulier trois encoches (27) régulièrement réparties sur la périphérie pour liaison à la manière d'une clé avec des tenons complémentaires d'un outil de direction (28).

13. Instrument auxiliaire selon l'une des revendications 1 - 12, caractérisé en ce que l'axe longitudinal (31) de l'ouverture traversante oblique (16) de la lame pour le col de fémur (19) fait un angle (α) de 125 degrés ou de 135 degrés avec l'axe longitudinal (32) de la section proximale (14) de l'aiguille de fixation (12).

14. Instrument auxiliaire pour ostéosynthèse selon l'une ou plusieurs des revendications précédentes, caractérisé en ce que la pièce pour col de fémur (19) présente en son extrémité externe opposée à la tête de fémur (18) une partie s'étendant radialement, en particulier un col (33) se prolongeant sur la périphérie, limitant l'engagement latéral de la pièce pour col de fémur (19) dans l'orifice traversant oblique (16) de l'aiguille de fixation (12).

15. Instrument auxiliaire selon la revendication 14, caractérisé en ce que l'extrémité frontale du côté du col de fémur (18), respectivement le bord frontal de la partie profilée (34), de la pièce pour col de fémur, en particulier de la lame pour col de fémur, (19) est formée en un ou plusieurs tranchants (37).

16. Instrument auxiliaire pour ostéosynthèse selon l'une ou plusieurs des revendications précédentes, caractérisé en ce qu'au moins l'un des perçages traversant dans la zone distale (15) de l'aiguille de fixation (12) est réalisé sous la forme d'un perçage traversant longitudinal (13), dans lequel peut être introduit un élément de fixation distal, en particulier un boulon de fixation distal, pour ancrage dans l'os du fémur, lequel boulon est introduit en l'extrémité proximale du perçage traversant longitudinal pour fixation statique distale de l'aiguille de fixation (12), et introduit en l'extrémité distale du perçage traversant longitudinal (13) pour fixation distale dynamique de l'aiguille de fixation (12), dans lequel le boulon de fixation distal présente un filetage auto-taraudant avec une profondeur minimale de filetage qui suffit juste encore pour retenir le boulon de fixation dans l'os du fémur.

17. Instrument auxiliaire selon la revendication 16, caractérisé en ce que le perçage traversant longitudinal (13) présente une longueur correspondant sensiblement au double du diamètre du boulon de fixation associé, respectivement de la largeur du perçage traversant longitudinal.

18. Instrument auxiliaire selon la revendication 16 ou 17, caractérisé en ce que le boulon de fixation distal peut être introduit au moyen d'un outil de direction (28) qui comprend une douille de direction (39) orientable sur 180 degrés et complétée d'un perçage (40) correspondant au diamètre du boulon de fixation afin de positionner le boulon de fixation dans le perçage traversant longitudinal (13).

19. Instrument auxiliaire (10) pour ostéosynthèse selon l'une ou plusieurs des revendications précédentes et comprenant un appareil de direction avec une douille de direction (39), caractérisé en ce que l'appareil de direction est verrouillable par un serrage par la forme et par la force en l'extrémité proximale (20) de l'aiguille de fixation (12) pour réalisation d'une liaison rigide, appareil comprenant également un bras (42) qui, en condition monté, se prolonge sensiblement parallèlement à l'aiguille de fixation (12) et en direction de l'extrémité distale et qui présente au moins un orifice longitudinal (38) associé à au moins un perçage traversant longitudinal (13) de la zone distale de l'aiguille de fixation (12) pour réception d'une douille de direction complémentaire (39), laquelle douille présente en l'une de ses extrémités un perçage de direction (40) dont le diamètre interne est un tout petit peu plus grand que le diamètre externe d'un boulon de fixation distal ou d'un foret d'os prévu pour être passé au travers, laquelle douille peut être tournée sur 180 degrés de telle sorte que le perçage de direction (40) peut être positionné en l'extrémité proximale ou distale du perçage traversant longitudinal (13) de la zone distale (15) de l'aiguille de verrouillage (12).
